(19) Europäisches Patentamt

European Patent Office

Office européen des brevets

(11) **EP 1 171 102 B1**

(12) **EUROPEAN PATENT SPECIFICATION**

(45) Date of publication and mention
of the grant of the patent:
**31.08.2005 Bulletin 2005/35**

(51) Int Cl.⁷: **A61K 9/36**, A61K 31/341,
C07D 307/52, A61P 1/04,
A61P 31/04

(21) Application number: **00925209.9**

(22) Date of filing: **11.04.2000**

(86) International application number:
**PCT/EP2000/003330**

(87) International publication number:
**WO 2000/061118 (19.10.2000 Gazette 2000/42)**

(54) **ANHYDROUS TABLET OF RANITIDINE HYDROCHLORIDE WITH DOUBLE-LAYER COATING AND ITS COMPOSITION**

WASSERFREIE RANITIDINHYDROCHLORID ENTHALTENDE TABLETTE MIT
ZWEISCHICHTIGEM ÜBERZUG UND DEREN ZUSAMMENSETZUNG

COMPRIME ANHYDRE D'HYDROCHLORURE DE RANITIDINE AVEC ENROBAGE DOUBLE
COUCHE ET SA COMPOSITION

(84) Designated Contracting States:
**AT BE CH CY DE DK ES FI FR GB GR IE IT LI LU
MC NL PT SE**

(30) Priority: **12.04.1999 IT MI990740**

(43) Date of publication of application:
**16.01.2002 Bulletin 2002/03**

(73) Proprietor: **Pharmexcel SRL
20144 Milano (IT)**

(72) Inventors:
• **VERONESI, Paolo, Alberto
I-20144 Milano (IT)**
• **PESCHECHERA, Emanuela
I-20133 Milano (IT)**

• **CIRERA DOTTI, Xavier
E-08950 Esplugues de Llobregat (ES)**

(74) Representative: **Pistolesi, Roberto et al
Dragotti & Associati srl
Via Turati 32
20121 Milano (IT)**

(56) References cited:
**EP-A- 0 694 540        WO-A-95/28918
DE-A- 19 511 131        US-A- 5 853 756**

Remarks:
The file contains technical information submitted
after the application was filed and not included in this
specification

EP 1 171 102 B1

**Description**

The invention concerns an anhydrous tablet containing anydrous ranitidine hydrochloride, coated by a double-layer coating, its composition and the relevant process of preparation.

The anhydrous tablet of the invention is characterized by the direct dry tabletting, without using water, of a mixture consisting of the active ingredient anhydrous ranitidine hydrochloride, in the crystalline form "allomorphous Form 1" having a particular purity and stability, and by a global amount of other excipients and adjuvant substances or inert vehicles less than the weight of the active ingredient. Moreover, the anhydrous tablet of the invention is characterized by a high percentage of disintegrating substance in the tabletting mixture and by a special two-layer coating.

In order to better illustrate, appreciate and characterize the complementary and synergic properties of the anhydrous tablets and of the other teachings of the invention as well as their intimate correlation, the next section "Publications" lists the most significant references concerning the state-of-the-art of ranitidine hydrochloride and its compositions. Ranitidine chemically identified with the IUPAC denomination N-[2-[[[5-[4-dimethylamino]methyl]-2-furanyl]methyl] thio]ethyl]-N'-methyl-2-nitro-1,1-etene diamine (6), is a well-known antagonist of $H_2$ histamine receptors widely employed in the medical practice as ranitidine hydrochloride for the treatment of gastric ulcers and of other gastric disorders and, recently, combined with other drugs for the eradication of *Helicobacter pylori*, since it is mainly administered by oral route and fomulated in conventional or coated tablets.

The Italian patent (1) (corresponding to priority GB 1,565,966), recognized to the British company Allen & Hamburys Limited, describes and claims simply the compound ranitidine hydrochloride without specifying any particular crystalline form. Few years later, the next Italian patent (2) (corresponding to priority GB 2,084,580), recognized to the British company Glaxo Group Limited, and also other later technical publications denote that ranitidine hydrochloride could exhibit the phenomenon of polymorphism, i.e. it could be available in two different crystalline forms: allomorphous Form 1, which can be obtained by the first procedure (1) and allomorphous Form 2, which is obtained by the procedure described in the second procedure (2). This latter, besides the procedure, claims mainly the crystalline form "Form 2". Moreover, the Italian patent N. 1.143.237 (2) claims the infrared spectrum and the scheme of diffraction at X rays, characteristic of Form 2 of ranitidine hydrochloride.

However, since polymorphism is a property only of the solid state and, accordingly, has no influence on the physical properties of ranitidine hydrochloride (5), such as its water solubility - since they are both the allomorphous freely soluble in water (900 mg in 1 ml of water) - it should be considered that the two allomorphous Form 1 and Form 2 exhibit identical pharmacological and clinical properties (Scrip n.° 2245, 1 July 1997, page 7 column 1, lines 40-45). However, it was ascertained that allomorphous ranitidine hydrochloride alone, Form 2, was for many years the single commercially available crystalline form (EP 0 626 381, page 2, line 19).

Moreover, the Italian patent N. 1.143.237 (2) teaches that ranitidine hydrochloride Form 1 is more hygroscopic than Form 2 and that, due to its more limited stability at room temperature, may be converted in small amounts in the presence of moisture into Form 2.

Publications

Publications are mentioned in the description with the same progressive number allotted in this list.

(1) Italian patent N. 1,126,759 (priority GB N. 1,565,966) recognized to Allen & Hamburys Ltd.;
(2) Italian patent No. 1,143,237 (priority GB N. 2,084,580) recognized to Glaxo Group Ltd.;
(3) EP No. 0 626 381 B1 recognized to Torcan Chem. Ltd.;
(4) US No. 4,672,133 recognized to Glaxo Group Ltd.;
(5) Chemical Abstracts, Vol. 96, No. 11, 1986, Columbus, Ohio, US; Abstract No. 51699z, Hohnjec, Marijan et al. "Physical and spectroscopic data of ranitidine";
(6) Chemical Abstracts, Vol. 104, No. 7, 1982, Columbus, Ohio, US; Abstract No. 88415p, "N-[2-[[[5-[4-dimethylamino] methyl]-2-furanyl]methyl]thio]ethyl]-N'-methyl-2-nitro-1,1-etene diamine";
(7) US 4,521,431 recognized to Glaxo Group Ltd.;
(8) US 5,663,381 recognized to Hexal Pharm., Inc.;
(9) US No. 4,880,636 A recognized to R. M. Franz;
(10) GB No. 2,229,094 A; Scrip n° 2245, 1 July 1997, page 7, column 1, lines 40 - 45;
(11) Drug Development and Industrial Pharmacy, Vol. 8, No. 3, 1982, pages 397-410, Marcel Dekker, Inc. New York, US; E.A. Gorman et al.: "An evaluation of croscarmellose as a tablet disintegrant in direct compression systems";
(12) Drug Development and Industrial Pharmacy, Vo. 17, No. 8, 1991, page 1067 - page 1081, Marcel Dekker, Inc. New York, US; K. Uzunarslanet et al.: "The effact of moisture on the physical characteristics of ranitidine hydrochloride tablets prepared by different binders and techniques";

(13) GB No. 2,218,336 B (priority of the application for patent US No. 194427 submitted on May 13,.1988) recognized to Glaxo Inc.;

(14) WO 95/15162 (priority DE P 43 41 310.2 submitted on December 03, 1993) recognized to Hexal Pharm., Inc.;

(15) WO 95/28918 (priority DK 0468/94 submitted on April 22, 1994) recognized to Gea Farmaceutisk Fabrik;

(16) Physicians' Desk Reference 41 st Ed., page 993 (1987);

(17) United States Pharmacopoeia XXIII Ed., page 1360;

(18) European Pharmacopoeia III Ed., page 1434;

(19) EP-0694540 filed by Ranbaxy Laboratories Ltd;

(20) US-5853756granted to J.B. Chemicals & Pharmaceuticals Ltd;

(21) DE-19511131filed by BASF AG.

The extraordinarily numerous publications, however, at the state-of-the-art, supply contradictory, obsolete data, which, however, are inadequate and insufficient to clear exhaustively the phenomenon of polymorphism of ranitidine hydrochloride and, moreover, the galenical compositions proposed for the preparation of tablets (14) meet in a very partial and unsatisfactory way the complex problems of technical character (12), necessary to obtain a coated tablet of ranitidine hydrochloride suitably formulated and stable.

The technical questions so far unsolved or without a satisfactory solution are summarized here below:

- allomorphous ranitidine hydrochloride of Form 2 is obtained from a reaction and a crystallization process (2), where ranitidine in the form of free base is made to react with hydrochloric acid in a hydroxylic solvent (propan-2-ol) and later is precipitated by crystallization after treatment with other hydroxylic solvents. Namely allomorphous ranitidine hydrochloride Form 2 may be obtained by dissolving ranitidine base in industrial methylated spirit (ethyl alcohol denaturated with methyl alcohol), containing about 2.0 % in volume of water, and "hydrogen chloride" (hydrochloric acid); afterwards crystallization is carried out at a high temperature, but not over 70° C;

- allomorphous ranitidine hydrochloride Form 1 obtained as described in patent (1) or in patent (8) exhibits no favorable filtration and good features of drying like Form 2 (US No. 4,672,133, column 1, lines 61-64);

- preparation of allomorphous ranitidine hydrochloride Form 2 requires necessarily the presence and/or the addition of 7.0 % and over of water to obtain the crystals of Form 2 (US No. 4,672,133, column 3, lines 49-51, column 4, lines 7-9, example 1, line 46, example 2, line 60, example 3, line 5, example 7, line 52, examples 10-12, lines 28-29, claim 6), in addition to the use of concentrated hydrochloric acid 35.0 % w/w in water and of industrial methylated spirit containing 2.0 % v/v of water (column 4, lines 38-40);

- moisture is partially eliminated from ranitidine hydrochloride Form 1 and also from Form 2 by drying the product under a reduced pressure at 50° C (4), but, of course, crystallization water, whenever present, cannot be removed at this low temperature (50° C) even operating at a reduced pressure;

- the above mentioned Pharmacopoeias (17, 18) establish that, for ranitidine hydrochloride, the maximum limit of moisture is 0.75%, which limit, however, is high for a product unstable in the presence of water;

- ranitidine hydrochloride Form 2 is less hygroscopic than Form 1 (US No. 4,672,133, column 1, lines 61-62);

- the British (2) and U.S. patents (4), from which ranitidine hydrochloride Form 2 is obtained, and the European patent (3), from which Form 1 is obtained, employ as solvent isopropanol, but in the first patent it is mentioned that necessarily water should be used to obtain the crystallization of Form 2, thus denoting indirectly that water is indispensable in Form 2 as crystallization water;

- in the Italian patent (1) for the allomorphous Form 1 and in the Italian patent (2) for the allomorphous Form 2 ranitidine hydrochloride "anhydrous" was not described and no limit of moisture or water content is taken into account for this substance;

- the sensitivity of ranitidine hydrochloride to moisture (US N. 4,421,431, column 1, lines 58-59) is widely described;

- ranitidine hydrochloride is subject to degradation in time and this process is favoured by heat, moisture and light (Physicians' Desk Reference, 41st ed., page 993, 1987 e GB 2,218,336, page 1, lines 25-28);

- ranitidine hydrochloride is not only soluble in water in appreciable amounts (GB No. 2,229,094, page 1, lines 28-29) (solubility at 25° C is over 900 mg/ml), but is also very soluble in the most commonly used organic solvents (6). This high solubility and the propension to the degradation in the presence of heat, moisture and light, cause the resulting difficulty in maintaining the pharmaceutical integrity of the tablets coated by a simple polymeric film-coating containing ranitidine hydrochloride (GB 2,218,336, page 1, lines 29-34);

- (19) discloses a process for the manufacture of form 1 ranitidine hydrochloride;

- (20) discloses controlled release oral formulations of ranitidine hydrochloride in the form of coated tablets and capsules;

- (21) discloses mechanically stable solid formulations containing at least one ctive principle and at least one polymer.

The references mentioned above widely illustrate the various problems concerning the need of obtaining the substance ranitidine hydrochloride nearly devoid of moisture, and thus "anhydrous", particularly suitable for the production, by direct dry tabletting (without the aid of water), of an anhydrous tablet of ranitidine hydrochloride, to be further protected from athmospheric humidity by a suitable coating.

Description of the invention

The invention is defined in the claims and concerns an integrated procedure of various operations tending globally to optimize the production of an anhydrous tablet, obtained by direct dry tabletting of a mixture of anhydrous ranitidine hydrochloride, allomorphous Form 1, of a particular purity and stability, later coated by a suitable double-layer coating. This procedure of the invention may be schematized and subdivided into the following essential steps:

1) preparation of anhydrous allomorphous ranitidine hydrochloride Form 1, of a particular purity and stability, to be used for the composition of the tabletting mixture, precipitated and crystallized without the use of water, even using totally anhydrous solvents;
2) preparation of the mixture consisting of anhydrous allomorphous ranitidine hydrochloride active substance Form 1 and by other process adjuvants or carriers, in which the percentage of the disintegrating agent is between 8.0% and 12.0% of the total inert portion, and later transformation, by direct dry tabletting, without any use of granulation water into anhydrous tablets;
3) application of a double-layer coating on the anhydrous tablets, carried out in two following steps, suitably studied to make the tablets particularly resistant to the atmospheric humidity and stable during their use.

The invention is also characterized by the fact that, though each of the three steps of the procedure is by itself a significant inventive jump versus the state-of-the-art, their combined use enables to obtain an anhydrous tablet of ranitidine hydrochloride, which, properly protected by a double-layer coating, exhibits a remarkable resistance to moisture and stability on ageing.

Indeed, it could be particularly difficult to obtain an anhydrous tablet, if the particular type of allomorphous anhydrous type of ranitidine hydrochloride, Form I of the invention, is not used. On the contrary, allomorphous Form 2 of ranitidine hydrochloride is obtained necessarily with the addition of water and, thus, retains remarkable amounts of crystallization water in the end product, which passes also in the next tablet.

Moreover, a further characteristic of the invention is that the direct tabletting procedure of the mixture containing minimum amounts of other adjuvants or inert carriers with a percentage of disintegrating agent (11) between 8.0 % and 12.0 % of the inert portion and a global moisture less than 1.0% is necessarily dry without the aid of water.

Finally, a further characteristic of the invention is that the core of the anhydrous tablet, obtained in this way, is adequately protected by a special type of double-layer coating suitably studied to prevent that the active substance of the tablet, being hygroscopic, draws inside moisture, thus causing both morphologic changes of the outer surface (cuts, swellings or clefts) and degradation processes which can be easily identified by remarkable changes of color (from white to various shades of brown). Anhydrous tablets of the invention surprisingly exhibit a satisfactory rate of dissolution and a very good stability, since they do not undergo degradation processes which may be shown by changes of color, though stored even for long periods under normal conditions of temperature and in conventional containers, such as glass vials sealed with a suitable stopper or blisters usually commercially available.

Moreover, the anhydrous tablet of the invention exhibits an acceptable organoleptic character (it has no bitter taste on the surface) and a satisfactory bioavailability.

To better illustrate the anhydrous tablet of ranitidine hydrochloride of the invention, we report here below in a more detailed form the various steps of the preparation.

Preparation of anhydrous allomorphous ranitidine hydrochloride Form 1 particularly pure and stable.

The first step concerns a procedure of manufacturing of anhydrous allomorphous ranitidine hydrochloride Form 1, particularly pure and stable, in order to meet the need of having at disposal an anhydrous active substance to produce the anhydrous tablet of the invention.

As reported above, allomorphous Form 2 is produced by making to react the free base with concentrated hydrochloric acid (about 35.0% in water) in an hydroxyl solvent, and the resulting ranitidine hydrochloride is crystallized by adding water in an amount greater than 7.0% and additional amounts of the same solvent. On the contrary, it was surprisingly discovered in the first step of the procedure of the invention that if ranitidine base, suspended uniformly in totally anhydrous ethyl acetate, is made to react under constant stirring with a solution of hydrochloric acid in anhydrous isopropanol at a concentration variable from 2.0 % to 12.0 %, added dropwise to the former one, ranitidine hydrochloride which is obtained by later precipitation consists of crystals of anhydrous allomorphous ranitidine hydrochloride, Form 1, particularly pure and stable. Crystals collected, after washing with a mixture of anhydrous ethyl acetate and isopropanol and later with anhydrous ethyl acetate alone, are released from the reaction solvents by means of traditional drying methods.

The study performed with the modern and complex techniques of NMR (*Nuclear Magnetic Resonance*) at the solid state, employed on the core of $^{13}$C, such as the *Cross Polarization - Magic Angle Spilling* (CP-MAS) and the *Total suppression spin side band* (CP-Toss) have supplied spectra $^{13}$C NMR (*$^{13}$C Nuclear Magnetic Resonance)* of the test sample which confirm that anhydrous ranitidine hydrochloride of the invention, compared with another sample of product obtained with the procedure of patent (2) (surely consisting of allomorphous ranitidine hydrochloride Form 2), on the contrary consists only of crystals of allomorphous ranitidine hydrochloride Form 1.

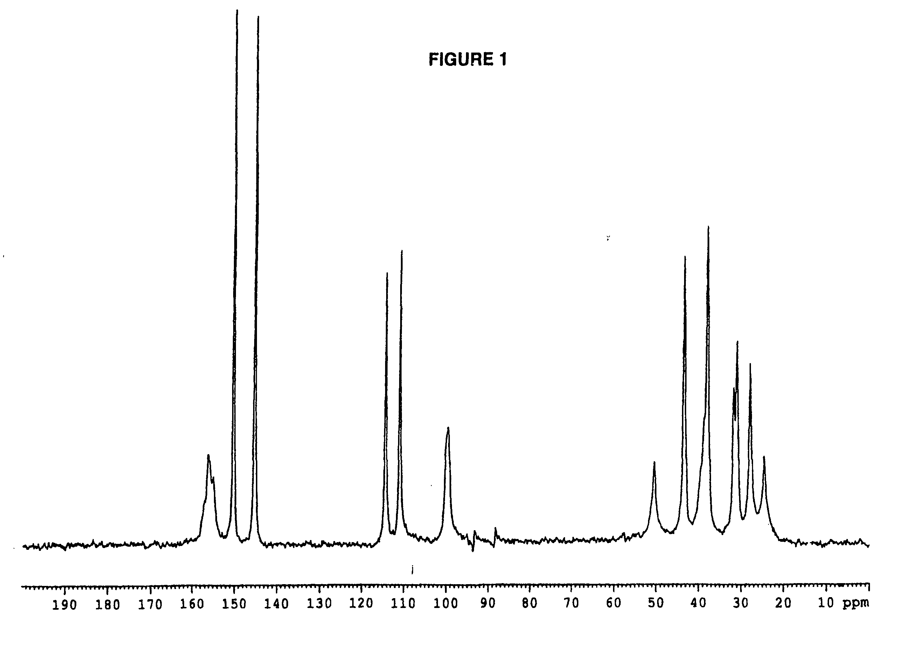

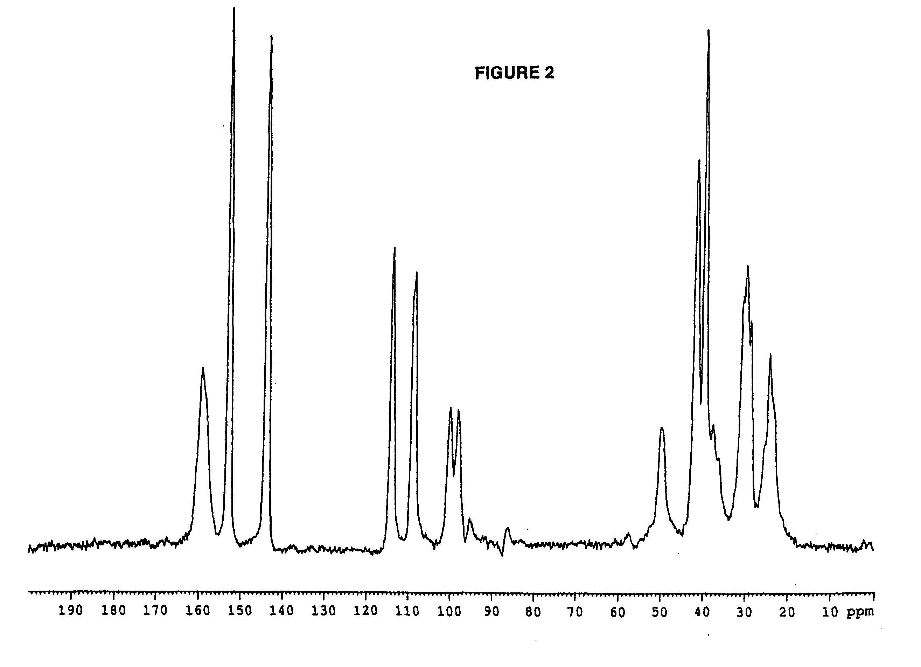

The main differences between the two allomorphous forms are obvious both in the spectrum region at about 110 ppm (see the spectra of $^{13}$C NMR enclosed herewith of allomorphous ranitidine hydrochloride Form 1 - Figure 1 and of allomorphous Form 2 - Figure 2), where variations of *chemical shift* are observed, and in the spectrum region at 100 ppm, where the signal appears to be split into the allomorphous Form 2. Moreover, anhydrous ranitidine hydrochloride of the invention shows at the origin a very negligible water content. Therefore, for this product, the maximum limit of acceptance of 0.3% was adopted, estimated by the micrometric method of Karl Fischer (European Pharmacopoeia III Ed., method 2.5.12, page 66), whereas the limit of the water content prescribed by the Phamacopoeias (17, 18) for ranitidine hydrochloride is 0.75%.

The procedure to obtain anhydrous ranitidine hydrochloride of the invention meets the requirement of an industrial production, since it enables to remove and to recover the reaction and catalyzation solvents which could be used again.

The drying operations are carried out by conventional equipment and methods, under a reduced pressure, preferably in an inert nitrogen atmosphere, in order to prevent the problems reported in the literature for ranitidine hydrochloride, resulting from the exposure to temperatures over 50° C and to atmospheric humidity.

Allomorphous Form I of anhydrous ranitidine hydrochloride, produced according to the method of the invention, exhibits good features of filtration and drying and is not gradually converted during the storage into the allomorphous Form 2. Therefore, it cannot be found in the end product, as it is demonstrated also by the chemico-physical analysis.

Preparation by direct (dry) tabletting of the anhydrous tablet of anhydrous allomorphous ranitidine hydrochloride Form 1 particularly pure and stable.

The second aspect peculiar to the present invention concerns the procedure to obtain the anhdyrous tablet by means of a direct dry tabletting, without using water, of the mixture of powders containing minimum amounts of inert adjuvant or carrying substances, as reported in the composition of the invention. Namely, the above mentioned substances consist of a disintegrating agent, such as croscarmellose sodium (also known with as reticulated carboxymethylcellulose sodium, since this product is commercially available with trade marks A-DI-C-SOL® and EXPLOTAB®), a binding agent such as microcrystalline cellulose (Avicel 101®), a lubricant, such as magnesium stearate, and a flowing agent, such as silica, which are mixed in the following proportions, expressed as percentage of the global weight of the inert portion of the tablet:

| Ingredients | percentage (%) |
|---|---|
| microcrystalline cellulose | 73.0 - 83.0 |
| croscarmellose sodium | 8.0 - 12.0 |
| magnesium stearate | 5.0 - 9.0 |
| silica | 4.0 - 6.0 |

The inert ingredients, in the most suitable proportions adopted, are transferred to a conventional mixer of powders, along with the necessary amount of anhydrous allomorphous ranitidine hydrochloride Form 1. The amount of active ingredient for each anhydrous tablet is preferably between 80.0 and 350.0 mg.

Another main feature of the anhydrous tablet of the invention is that the active ingredient anhydrous ranitidine hydrochloride is at least the 50.0% of the global weight of the anhydrous tablet and that the disintegrating agent croscarmellose sodium is at least 8.0 % of the inert component of the tablet.

The anhydrous tablet of the invention is obtained by direct dry tabletting, without the addition of water, to the mixture of powder, according to the well-known conventional techniques of the pharmaceutical industry for the production of tablets.

Application of the double-layer coating of the anhydrous tablet of ranitidine hydrochloride.

The third main feature of the present invention consists in the application of a double-layer coating on the anhydrous tablets and in the relevant procedure.

In general, the film-coating of the tablets is obtained either by sugar-coating or with techniques of coating in which the film-coating mixture, dissolved in water or in organic solvents, is applied with a procedure of continuous automatic spraying using a pressurized air flow which fixes the coating to the surface of the tablet. The coating, besides organoleptic (removal of unplcasant tastes or odors) or cosmetic reasons (improvement of color or aspect), is also adopted to protect the active ingredient contained in the tablet from atmospheric humidity and/or from light.

Indeed, a suitable coating is capable to better protect the product and to delay during its storage the degradation processes which may be shown by a reduction of the activity of the drug and possible changes of color of the tablet surface.

However, it is fundamental that the tablet coating does not shorten the disgregation time of the tablet and, therefore, does not influence negatively the bioavailability of the active ingredient contained.

In view that many authors have reported that ranitidine hydrochloride has the propension during the storage of undergoing a degradation in the presence of heat, moisture and light, causing not only a reduction of activity, but also marked phenomena of color on the tablet surface, it is necessary that the coating adopted combines the requirements and the features mentioned above. Moreover, anhydrous ranitidine hydrochloride has properties which make rather difficult the coating of the anhydrous tablet, since this should be rather thick to protect effectively the active ingredient contained, but without reducing the dissolution and bioavailability of the drug. Because ranitidine hydrochloride is highly soluble in water and in many other organic solvents, it could easily migrate through the coating (applied with water or organic solvents) during the drying step and also later, facilitated by atmospheric humidity. The amount of ranitidine hydrochloride which penetrates or migrates through the coating, is subject to a fast staining under the influence of light and moisture (WO 95/28918, page 2, lines 24-32).

These technical problems concerning the coating of the anhydrous tablet may be partly reduced with the use of anhydrous allomorphous ranitidine hydrochloride Form I pure and stable of the invention, but the conventional coatings could only partially obviate the mentioned problems of stability, in particular the problem of the dark staining of the surface appearing already after few months. Some solutions to reduce the above mentioned problems concerning the tablets containing common ranitidine hydrochloride were described in the past by some authors, but they were found to be insufficient, since the normal commercially available tablets of ranitidine hydrochloride have a shelf-life of only 24 months, in view that the degradation processes could appear significantly only after 24 months of storage.

In the known practice concerning the coating of normal tablets of ranitidine hydrochloride obtained from (1) or (2), coatings were used with polymeric films combined with plasticizing agents (e.g. triacetin) alone or mixed in different proportions. However, some conventional polymeric coatings are incompatible with ranitidine hydrochloride and thus accelerate its degradation, Indeed, it was reported in some publications that many plasticizing agents employed usually in the polymeric coatings of pharmaceutical products, as an example polyethylene glycol, propylene glycol, mineral oil, are unacceptable and, thus, unusable if applied directly in contact with the surface of the tablets of ranitidine hydrochloride (GB 2 218 336, page 2, lines 1 - 25). However, these plasticizing agents should be necessarily added to the polymeric solution or suspension in order to improve the elasticity of the coating and minimizing the risks of cuts or swellings of clefts on the surface of tablets coated during the storage (GB 2 218 336, page 2, lines 1 - 6). Moreover, it would particularly preferable to use for the tablet coating a mixture of film-coating agents in an aqueous solution or suspension, in order to prevent the serious problems related to safety (antideflagrant plants to prevent risks of explosions and damages for the health of the operators) and ecology (elimination and/or recovery of the organic solvents employed) resulting from the use of organic solvents.

More in detail, it was surprisingly discovered in the procedure of the invention that a suitable and satisfactory coating for the anhydrous tablet of allomorphous anhydrous ranitidine hydrochloride, Form 1, pure and stable, consists of two layers applied overlapping and having different compositions. More in detail, it was discovered that, to solve the above mentioned problems, it is necessary that the coating of the anhydrous tablet of the invention is formed by a first inner layer consisting of hydroxypropylmethyl cellulose (HPMC) type E5, in amounts ranging from 5.0 and 15.0 mg, and by a second layer applied onto the first one, consisting of a mixture of hydroxypropylmethyl cellulose (HPMC) type E5, in amounts ranging from 5.0 and 25.0 mg, talc, in amounts ranging from 0.5 and 3.0 mg, titanium dioxide, in amounts ranging from 2.0 and 8.0 mg, and polyethylene glycol 4000-6000, in amounts ranging from 1.0 and 5.0 mg. Moreover, the weight of the coating should not be less than 4.5 % of the global weight of the anhydrous tablet and the ratio between the external and internal amount of hydroxypropylmethyl cellulose should be between 1:1 and 1:1.7. The two layers of the coating are applied using small amounts of water as a suspending vehicle, which is removed during the coating process.

The invention, moreover, is better illustrated in the following examples, which, however, are not a limit of the substance of the invention itself.

Example 1

Preparation of anhydrous allomorphous ranitidine hydrochloride, Form 1, particularly pure and stable.

Under conditions of inert nitrogen-saturated atmosphere, 10.0 g of ranitidine base are suspended in 100 ml of anhydrous ethyl acetate (moisture < 0,01 %). To the mixture, 21.95 g of hydrochloric acid in isopropanol are added dropwise (concentration 5.29 % mol/mol), during 45 minutes, keeping the temperature at 25° C. Stirring vigorously, again under inert nitrogen atmosphere, a white precipitate is obtained. Operating again in a nitrogen-saturated atmosphere, the precipitate is washed and filtered with a mixture of ethyl acetate/isopropanol in a ratio of 80:20 and afterwards with ethyl acetate alone. The resulting precipitate is dried in a oven at 40°- 50° C to remove the residual solvents and afterwards is placed in a desiccator *in-vacuo* on phosphorus pentoxide, until a constant weight is obtained. The moisture of the resulting product is less than 0.3 %. Analysis NMR-$^{13}$C at the solid state, carried out on the powder as such, confirms that the resulting ranitidine hydrochloride anhydrous contains exclusively allomorphous Form 1, since no traces of allomorphous Form 2 are present.

Global yield of the procedure: 91.7 %.

Example 2

Preparation of 25,000 anhydrous tablets containing 335 mg of anhydrous allomorphous ranitidine hydrochloride, Form 1.

All the steps of manufacturing are carried out in environments with relative moisture in an interval between 35 % and 50 % and at a temperature between 19° and 25° C, far from the light.

Qualitative and quantitative composition of the anhydrous tablet of the invention :

| Ingredients | mg/anhydrous tablet |
|---|---|
| anhydrous ranitidine hydrochloride allomorphous Form 1 (*) | 334.80 |
| microcrystalline cellulose (Avicel 101®) | 213.20 |
| croscarmellose sodium | 30.00 |
| magnesium stearate | 20.00 |
| silica | 12.00 |
| Total weight | 610.00 |

(*) equivalent to 300 mg of ranitidine

Amounts necessary for the preparation of 25,000 anhydrous tablets:

| Ingredients | Kg |
|---|---|
| anhydrous ranitidine hydrochloride allomorphous Form I (*) | 8.370 |
| microcristalline cellulose (Avicel 101®) | 5.330 |
| croscarmellose sodium | 0.750 |
| magnesium stearate | 0.500 |
| silica | 0.300 |

Into a suitable mixer for powders introduce in succession 5.330 Kg of microcrystalline cellulose (Avicel 101®), 0.500 Kg of magnesium stearate, 0.300 Kg of silica, 8.370 Kg of allomorphous ranitidine hydrochloride, Form 1, and, finally, 0.750 Kg of croscarmellose sodium. Moreover, it is checked that the global moisture of the mixture of powders is less than 1.0 %, so that the resulting tablet is virtually anhydrous. Mix the mixture of powders till homogeneity. The resulting mixture of powders is dry sieved and directly tabletted, without the aid of granulation water, with a conventional tabletting machine with an oblong punch, in the form of a bomb-shaped, capsule of 19 x 7 mm, with breaking sign on the upper punch, to obtain grooved tablets. Each resulting anhydrous tablet has a unit weight in the interval between 579.5 and 640.5 mg (± 5 %).

The resulting tablets, after the necessary analytical controls, are transferred to the next coating process. Analysis NMR-$^{13}$C, performed by the techniques CP-MAS and CP-Toss on the anhydrous tablet pulverized in a mortar, confirmed that it contains only allomorphous ranitidine hydrochloride, Form 1.

From the tabletting process described, 24,178 anhydrous tablets of anhydrous ranitidine hydrochloride are obtained.

Global yield of the procedure: 96.7 %

Example 3

Application of a double-layer coating to 24,000 anhydrous tablets of anhydrous allomorphous ranitidine hydrochloride, Form 1.

The anhydrous tablets produced with the method described in Example 2 are subjected to the next step of double-layer coating.

Qualitative and quantitative composition of the double-layer coating of each ahydrous tablet:

| First layer: | |
| --- | --- |
| Ingredient | Amount (mg) |
| Hydroxypropylmethyl cellulose (Type E5) | 10.0 |

| Second layer: | |
| --- | --- |
| Ingredient | Amount (mg) |
| Hydroxypropylmethyl cellulose (type E5) | 13.0 |
| Polyethylene glycol 6000 | 2.0 |
| Talc | 1.0 |
| Titanium dioxide | 4.0 |
| Total weight of the coating | 30.0 |

Amounts necessary for the double-layer coating of 24,000 anhydrous tablets :

| First layer: | |
| --- | --- |
| Ingredient | Amount (grams) |
| Hydroxypropylmethyl cellulose (type E5) | 240.0 |

| Second layer : | |
| --- | --- |
| Ingredient | Amounts (grams) |
| Hydroxypropylmethyl cellulose (type E5) | 312,0 |
| Polyethylene glycol 6000 | 48.0 |
| Talc | 24.0 |
| Titanium dioxide | 96.0 |

For the preparation of each suspension to be applied as first and second layer, 20.0% and 16.0% aqueous mixtures are prepared respectively.

For the preparation and application of the first layer, 240.0 g of hydroxypropylmethyl cellulose are mixed with a suitable amount of water, until a homogeneous suspension is obtained. For the preparation of the second layer, 312.0 g of hydroxypropylmethyl cellulose (type E5), 48.0 g of polyethylene glycol 6000, 24.0 g of talc and 96.0 g of titanium dioxide are mixed with a suitable amount of water until a homogeneous suspension is obtained.

The coating of the tablets is applied to the anhydrous tablets in two separate steps, by the conventional methods well known by the experts of this field, more specifically by spraying on the tablets the film-coating mixtures and simultaneously exposing them to the drying compressed air in suitable revolving pans.

Global yield of the procedure: 96.06 %.

Example 4

Stability study of coated anhydrous tablets of the invention containing anhydrous allomorphous ranitidine hydrochloride, Form 1.

The coated tablets of the invention, subjected to a stability study lasting 36 months, during which they were stored far from light in suitable blisters (coupled sheets of PVC/PVDC opacified with titanium dioxide - Mark Sicovimp[R] 201103), at room temperature (25° C) and at relative humidity of 65 %, supplied the results shown in the next Tables 1, 2 e 3.

During the stability study, the following parameters of the anhydrous coated tablet of the invention were assessed:

| Parameters | Limits |
|---|---|
| * Appearannce | White tablet |
| * Identification (HPLC) | Positive |
| (anhydrous allomorphous ranitidine hydrochloride, Form 1) | |
| * Dissolution | ≥ 80.0 % in 45 minutes |
| * Assay % (HPLC) | 90.0 % - 110.0 % of the |
| (anhydrous allomorphous ranitidine hydrochloride, Form 1) | labelled content |

Controls were carried out every 12 months from the date of preparation. The results obained clearly demonstrate that the coated anhydrous tablets of the invention, containing anhydrous allomorphous ranitidine hydrochloride, Form 1, suitably packaged in blisters, exhibit a good stability when stored at room temperature (25° C) and under normal conditions of relative humidity.

Indeed, during the storage period of 36 months, the aspect of the tablets remained unchanged, dissolution underwent no significant variations and the HPLC analysis showed only moderate processes of degradation of the active ingredient, which, however, remained within the limits adopted.

TABLE 1

| Batch No. SP-94-03 | Mfg date: March 7, 1994 | | | | |
|---|---|---|---|---|---|
| Parameters | Limits | Beginning | 12 months | 24 months | 36 months |
| | | 7-03-94 | 15-03-95 | 29-03-96 | 13-03-97 |
| Appearance | White tablets | Conf. | Conf. | Conf. | Conf. |
| Identification (HPLC) | Positive | Pos. | Pos. | Pos. | Pos. |
| Dissolution | ≥80.0 % | 93.4 | 94.7 | 92.1 | 93.9 |
| Assay (HPLC) | 90 - 110% | 101.2 | 100.1 | 98.8 | 98.1 |
| Conf. = Conform Pos. = Positive | | | | | |

TABLE 2

| Batch No. SP-94-07 | Mfg date: July 4, 1994 | | | | |
|---|---|---|---|---|---|
| Parameters | Limits | Beginning | 12 months | 24 months | 36 months |
| | | 4-07-94 | 19-07-95 | 11-07-96 | 02-07-97 |
| Appearance | White tablets | Conf. | Conf. | Conf. | Conf. |
| Identification (HPLC) | Positive | Pos. | Pos. | Pos. | Pos. |
| Dissolution | ≥80,0% | 91.7 | 93.4 | 91.2 | 90.7 |
| Assay (HPLC) | 90 - 110% | 100.3 | 99.7 | 99.2 | 98.7 |
| Conf. = Conforme Pos. = Positive | | | | | |

TABLE 3

| Batch No. SP-94-11 | Mfg date: November 3, 1994 | | | | |
|---|---|---|---|---|---|
| **Parameters** | **Limits** | **Beginning** | **12 months** | **24 months** | **36 months** |
| | | 3-11-94 | 09-11-95 | 21-11-96 | 18-11-97 |
| Appearance | White tablets | Conf. | Conf. | Conf. | Conf. |
| Identification (HPLC) | Positive | Pos. | Pos. | Pos. | Pos. |
| Dissolution | $\geq$80.0 % | 94.2 | 92.5 | 91.4 | 92.3 |
| Assay (HPLC) | 90 - 110 % | 100.9 | 99.8 | 99.1 | 98.0 |
| Conf. = Conform Pos. = Positive | | | | | |

**Claims**

1. A composition, for preparing an anhydrous coated tablet by direct compression, comprising a mixture of anhydrous ranitidine hydrochloride, allomorph Form 1, and inert diluents, which mixture contains less than 1 % water.

2. A method of preparing a composition for an anhydrous tablet as claimed in claim 1, comprising:

   a) preparing anhydrous ranitidine hydrochloride, allomorph Form 1, without the use of water, in totally anhydrous organic solvents;
   b) preparing an anhydrous tablet by dry direct tabletting of the anhydrous ranitidine hydrochloride, allomorph Form 1, wherein the anhydrous ranitidine hydrochloride is at least the 50 % of the global weight of the tablet and the global moisture content of the tablet is less than 1.0 % water; and
   c) applying on the surface of the anhydrous tablet, with consecutive and independent processes two layers coating having different compositions, being the global weight of the double-layer coating not less than 4.5% of the total weight of the anhydrous tablet.

3. A composition as claimed in claim 1 wherein the dry direct tabletting is carried out with a mixture containing anhydrous ranitidine hydrochloride allomorph Form 1 admixed with inert excipients in the following ratios, expressed as percentages of the global weight of the inert portion of the tablet:

| microcrystalline cellulose | 73.0 % - 83.0 % |
|---|---|
| croscarmellose sodium | 8.0 % - 12.0 % |
| magnesium stearate | 5.0 % - 9.0 % |
| silica | 4.0%-6.0% |

4. A process as claimed in claim 2 wherein anhydrous ranitidine hydrochloride, allomorph Form 1, having a moisture less than 0.3% water, is obtained from the reaction of ranitidine base, uniformly suspended in totally anhydrous ethyl acetate, with a solution of hydrogen chloride in anhydrous isopropanol in a percentage amounts of from 2.0 % to 12.0 %, added dropwise and under an inert nitrogen atmosphere at a temperature of about 25° C, followed by precipitation and crystallization, without addition of water.

5. A process as claimed in claim 2 wherein the double-layer coating on the surface of the anhydrous tablet consists of an inner layer of hydroxypropylmethyl cellulose alone in an amount ranging from 5.0 and 15.0 mg and an outer layer containing a mixture of hydropropylmethyl cellulose in amount ranging from 5.0 and 25.0 mg, talc in amount ranging from 0.5 and 3.0 mg, titanium dioxide in amount ranging from 2.0 and 8.0 mg and polyethylene glycol 4000-6000 in amount ranging from 1.0 and 5.0 mg, wherein the ratio of the amounts of hydroxypropylmethyl cellulose in the inner and outer layers ranges from 1 : 1 to 1 : 1.7.

6. A pharmaceutical composition comprising an anhydrous tablet of anhydrous ranitidine hydrochloride, allomorph Form 1, when made by a process as claimed in any one of claims 2, 4 and 5.

7. Use of a pharmaceutical composition as claimed in any one of claims 1, 3 and 6 for preparing direct compression anhydrous tablet, containing anhydrous ranitidine hydrochloride, allomorph Form 1, for the oral treatment of ulcers and of other gastric disorders and for the eradication of *Helicobacter pylori.*

**Patentansprüche**

1. Zusammensetzung zur Erzeugung einer wasserfreien beschichteten Tablette durch direkte Kompression, umfassend eine Mischung aus wasserfreiem Ranitidinhydrochlorid mit der allomorphen Form 1 und inerte Verdünnungsmittel, wobei die Mischung weniger als 1 % Wasser enthält.

2. Verfahren zur Erzeugung einer Zusammensetzung für eine wasserfreie Tablette wie in Anspruch 1 beansprucht, umfassend

    a) Herstellung von wasserfreiem Ranitidinhydrochlorid in der allomorphen Form 1 ohne Verwendung von Wasser in vollständig wasserfreien organischen Lösungsmitteln;

    b) Herstellung einer wasserfreien Tablette durch trockenes direktes Tablettieren des wasserfreien Ranitidinhydrochlorides mit der allomorphen Form 1, worin das wasserfreie Ranitidinhydrochlorid zumindest 50 % des Gesamtgewichtes der Tablette ausmacht und der gesamte Feuchtigkeitsgehalt der Tablette weniger als 1,0 % Wasser ist; und

    c) Auftragung einer zweischichtigen Beschichtung mit unterschiedlichen Zusammensetzungen auf die Oberfläche der wasserfreien Tablette mit unterschiedlichen Zusammensetzungen durch aufeinanderfolgende und unabhängige Verfahren, wobei das Gesamtgewicht der doppelschichtigen Beschichtung nicht weniger als 4,5 % des Gesamtgewichtes der wasserfreien Tablette ausmacht.

3. Zusammensetzung nach Anspruch 1, worin das direkte trockene Tablettieren mit einer Mischung mit wasserfreiem Ranitidinhydrochlorid in der allomorphen Form 1 durchgeführt wird, die mit inerten Exzipienten in den folgenden Verhältnissen vermischt ist, ausgedrückt als Prozentsätze des Gesamtgewichtes des inerten Anteils der Tablette:

| | |
|---|---|
| mikrokristalline Zellulose | 73,0 % - 83,0 % |
| Krosskaramellose-Natrium | 8,0 % - 12,0 % |
| Magnesiumstearat | 5,0 % - 9,0 % |
| Silica | 4,0 % - 6,0 % |

4. Verfahren nach Anspruch 2, worin wasserfreies Ranitidinhydrochlorid mit der allomorphen Form 1 mit einem Feuchtigkeitsgehalt von weniger als 0,3 % Wasser erhalten wird von der Reaktion einer Ranitidinhbase, die gleichmäßig in vollständig wasserfreiem Ethylacetat suspendiert ist, mit einer Lösung aus Chlorwasserstoff in wasserfreiem Isopropanol in einer Menge von 2,0 bis 12,0 %, die tropfenweise und unter einer inerten Stickstoffatmosphäre bei einer Temperatur von etwa 25°C zugegeben wird, mit anschließendem Ausfällen und Kristallisieren ohne Zugabe von Wasser.

5. Verfahren nach Anspruch 2, worin die doppelschichtige Beschichtung auf der Oberfläche der wasserfreien Tablette aus einer inneren Schicht aus Hydroxypropylmethylzellulose allein in einer Menge im Bereich von 5,0 bis 15,0 mg und einer äußeren Schicht mit einer Mischung aus Hydroxypropylmethylzellulose in einer Menge im Bereich on 5,0 und 25,0 mg, Talkum in einer Menge von 0,5 bis 3,0 mg, Titandioxid in einer Menge von 2,0 bis 8,0 mg und Polyethylenglycol 4000-6000 in einer Menge von 1,0 bis 5,0 mg besteht, wobei das Verhältnis der Mengen an Hydroxypropylmethylzellulose in der inneren und äußeren Schicht im Bereich von 1 : 1 bis 1 : 1,7 liegt.

6. Pharmazeutische Zusammensetzung, umfassend eine wasserfreie Tablette aus wasserfreiem Ranitidinhydrochlorid in der allomorphen Form 1, erzeugt durch ein Verfahren nach einem der Ansprüche 2, 4 und 5.

7. Verwendung einer pharmazeutischen Zusammensetzung wie in einem der Ansprüche 1, 3 und 6 beansprucht, zum direkten Komprimieren einer wasserfreien Tablette, umfassend wasserfreies Ranitidinhydrochlorid in der allomorphen Form 1, für die orale Behandlung von Geschwüren und anderen Magenerkrankungen und zum Auslöschen von Helicobacter Pylori.

## EP 1 171 102 B1

**Revendications**

1. Composition pour préparer un comprimé enrobé anhydre par compression directe, comprenant un mélange de chlorhydrate de ranitidine anhydre, forme 1 allomorphe, et de diluants inertes, lequel mélange contient moins de 1% d'eau.

2. Procédé de préparation d'une composition pour un comprimé anhydre selon la revendication 1, comprenant les étapes consistant à :

   a) préparer du chlorhydrate de ranitidine anhydre, forme 1 allomorphe, sans utiliser d'eau, dans des solvants organiques totalement anhydres ;

   b) préparer un comprimé anhydre par compression directe à sec du chlorhydrate de ranitidine anhydre, allomorphe de forme 1, le chlorhydrate de ranitidine anhydre représentant au moins 50% du poids total du comprimé et la teneur totale en humidité du comprimé étant inférieure à 1,0% d'eau ; et

   c) appliquer à la surface du comprimé anhydre, avec des procédés consécutifs et indépendants, deux couches d'enrobage ayant des compositions différentes, le poids total de l'enrobage double couche n'étant pas inférieur à 4,5% du poids total du comprimé anhydre.

3. Composition selon la revendication 1, dans laquelle la compression directe à sec est effectuée avec un mélange contenant du chlorhydrate de ranitidine anhydre, forme 1 allomorphe, associé à des excipients inertes selon les taux suivants, exprimés en pourcentage du poids total de la partie inerte du comprimé :

| | |
|---|---|
| cellulose microcristalline | 73,0% - 83,0% |
| croscarmellose sodique | 8,0% - 12,0% |
| stéarate de magnésium | 5,0% - 9,0% |
| silice | 4,0% - 6,0% |

4. Procédé selon la revendication 2, dans lequel le chlorhydrate de ranitidine anhydre, forme 1 allomorphe, ayant une humidité inférieure à 0,3% d'eau, est obtenu par la réaction de ranitidine base, mise en suspension de manière homogène dans de l'acétate d'éthyle totalement anhydre, avec une solution de chlorure d'hydrogène dans de l'isopropanol anhydre en des quantités, en pourcentage, de 2,0% à 12,0%, ajoutée au goutte à goutte et sous atmosphère d'azote inerte à une température d'environ 25°C, suivie d'une précipitation et d'une cristallisation, sans ajout d'eau.

5. Procédé selon la revendication 2, dans lequel l'enrobage double couche à la surface du comprimé anhydre est constitué d'une couche interne d'hydroxypropylméthylcellulose seule en une quantité dans la plage de 5,0 à 15,0 mg et d'une couche externe contenant un mélange d'hydroxypropylméthylcellulose en une quantité dans la plage de 5,0 à 25,0 mg, de talc en une quantité dans la plage de 0,5 à 3,0 mg, de dioxyde de titane en une quantité dans la plage de 2,0 à 8,0 mg et de polyéthylène glycol 4000-6000 en une quantité dans la plage de 1,0 à 5,0 mg, le rapport des quantités d'hydroxypropylméthylcellulose dans les couches interne et externe se situant dans la plage de 1 / 1 à 1/1,7.

6. Composition pharmaceutique comprenant un comprimé anhydre de chlorhydrate de ranitidine anhydre, forme 1 allomorphe, préparée par un procédé selon l'une quelconque des revendications 2, 4 et 5.

7. Utilisation d'une composition pharmaceutique selon l'une quelconque des revendications 1, 3 et 6 pour préparer un comprimé anhydre par compression directe, contenant du chlorhydrate de ranitidine anhydre, forme 1 allomorphe, destiné au traitement oral des ulcères et d'autres troubles gastriques et à l'élimination de *Helicobacter pylori*.

FIGURE 1

FIGURE 2